# EUROPEAN PATENT SPECIFICATION

(11) **EP 1 618 880 B1**
(45) Date of publication and mention of the grant of the patent: **14.07.2010**
(21) Application number: 04380155.4
(22) Date of filing: 20.07.2004
(51) Int. Cl.: A61K 31/352, A61K 47/10, A61K 47/02, A61P 29/00

(54) **Use of compounds derived from 2,3-dehydronaringenin for the treatment of inflammatory processes and pharmaceutical composition containing said derivatives**
Verwendung von 2,3-Dehydronaringenin-Derivaten zur Behandlung von Entzündungsprozessen und diese Derivate enthaltende pharmazeutische Zusammensetzung
Utilisation de dérivés de la 2,3-déhydronaringénine dans le traitement des processus inflammatoires et composition pharmaceutique comprenant ces dérivés

(43) Date of publication of application: 25.01.2006
(73) Proprietor: Furfural Espanol, S.A., 30820 Alcantarilla, Murcia (ES)
(72) Inventor: Lorente Salinas, Juan, 30002 Murcia (ES); Castillo Sanchez, Julian, 30011 Murcia (ES); Benavente-Garcia Garcia, Obdulio, 30100 Murcia (ES); Vicente Ortega, Vicente, 30008 Murcia (ES); Yanez Gascon, Josefa, 30012 Murcia (ES); Solano Munoz, Francisco, 3007 Murcia (ES); Alcaraz Banos, Miguel, 30011 Murcia (ES); Garcia Borron, Jose Carlos, 30004 Murcia (ES); Lozano Teruel, Jose Antonio, 30500 Monlina de Segura (ES)
(74) Representative: Carvajal y Urquijo, Isabel

(56) References cited:
- EP-A- 0 633 022
- US-A- 5 858 371
- DATABASE WPI Section Ch, Week 199626 Derwent Publications Ltd., London, GB; Class B04, AN 1996-255073 XP002309941 & JP 08 104628 A (SUMITOMO SEIYAKU KK) 23 April 1996 (1996-04-23)
- DATABASE CA [Online] CHEMICAL ABSTRACTS SERVICE, COLUMBUS, OHIO, US; KIM, CHANG JOHNG ET AL: "Pharmacological activities of flavonoids. (II). Relationships of anti-inflammatory and antigranulomatous actions" XP002309940 retrieved from STN Database accession no. 1991:464237 & YAKHAK HOECHI , 34(6), 407-14 CODEN: YAHOA3; ISSN: 0513-4234, 1990,
- GERRITSEN M E ET AL: "FLAVENOIDS INHIBIT CYTOKINE-INDUCED ENDOTHELIAL CELL ADHESION PROTEIN GENE EXPRESSION" AMERICAN JOURNAL OF PATHOLOGY, PHILADELPHIA, PA, US, vol. 147, no. 2, 1 August 1995 (1995-08-01), pages 278-292, XP000575649 ISSN: 0002-9440
- KIM H K ET AL: "ANTIINFLAMMATORY ACTIVITY OF FLAVONOIDS: MOUSE EAR EDEMA INHIBITION" ARCHIVES OF PHARMACAL RESEARCH, NATL. FISHERIES UNIVERSITY, PUSAN, KR, vol. 16, no. 1, 1993, pages 18-24, XP000886582 ISSN: 0253-6269
- LOGGIA R D ET AL: "THE ROLE OF FLAVONOIDS IN THE ANTIINFLAMMATORY ACTIVITY OF CHAMOMILLA RECUTITA" PROGRESS IN CLINICAL AND BIOLOGICAL RESEARCH, NEW YORK, NY, US, 1986, pages 481-484, XP001122218 ISSN: 0361-7742
- FOURIE, THEUNIS G. ET AL: "A flavone with antiinflammatory activity from the roots of Rhus undulata" JOURNAL OF NATURAL PRODUCTS , 47(6), 1057-8 CODEN: JNPRDF; ISSN: 0163-3864, 1984, XP008039862
- LIANG Y-C ET AL: "SYPPRESSION OF INDUCIBLE CYCLOOXYGENASE AND INDUCIBLE NITRIC OXIDE SYNTHASE BY APIGENIN AND RELATED FLAVONOIDS IN MOUSE MACROPHAGES" CARCINOGENESIS, XX, XX, vol. 20, no. 10, 1999, pages 1945-1952, XP002936369
- SONG JIN LEE ET AL: "ANTIINFLAMMATORY ACTIVITY OF NATURALLY OCCURRING FLAVONE AND FLAVONOL GLYCOSIDES" ARCHIVES OF PHARMACAL RESEARCH, NATL. FISHERIES UNIVERSITY, PUSAN, KR, vol. 16, no. 1, March 1993 (1993-03), pages 25-28, XP008001960 ISSN: 0253-6269

## Description

### FIELD OF THE INVENTION

The present invention is encompassed within the field of drugs for the treatment of inflammatory processes; it specifically refers to the use of 2,3-dehydronaringenin (apigenin) derivatives, of Formula (I), for the treatment or prophylaxis of inflammatory processes and chronic diseases derived from inflammatory processes, as well as to a pharmaceutical composition containing them, together with excipients.

### STATE OF THE ART

One of the major health problems in the current society is related to the inflammatory processes and diseases derived therefrom due to the discomforts and pain they cause in the patient. A classic example of this type of diseases related to inflammatory processes is rheumatoid arthritis (hereinafter, RA). The treatment of rheumatoid arthritis with drugs includes two large groups of drugs: one of them encompasses those serving to relieve the pain and inflammation on a short-term basis. They are useful for decreasing the inflammation and overcoming the "day-to-day" pain, but they do not serve for modifying the evolution of the disease on a long-term basis. The so-called non-steroidal anti-inflammatory drugs (NSAIDs) and corticoids are included in this group.

NSAIDs are cyclooxygenase inhibitors. The most frequent side effects of NSAIDs, gastrointestinal side effects, are related to their acidic nature and to prostaglandin inhibition. They can be expressed by different lesions, ranging from gastritis to ulcers, preferably gastric. These lesions can be asymptomatic or can be manifested with microscopic bleeding or clinically evident digestive hemorrhage. Risk factors for showing these complications are an elderly age and a prior history of epigastralgia or peptic ulcer. It is recommendable to take a gastric cytoprotective agent in these patients, and it is necessary to observe the occurrence of unusual digestive discomforts or changes in the deposition suggestive of digestive bleeding.

NSAIDs can cause a creatinine clearance decrease, by affecting the vasodilator function of prostaglandins. This is particularly important in elderly patients and in those with previously damaged renal function. Patients can develop acute interstitial nephropathy, papillary necrosis, and rarely, nephrotic syndrome. Secondary cutaneous, hepatic, hematological or neurological effects are less frequent. The choice of a specific NSAID is empirical and must be based on the pharmacokinetics of each preparation, including its half-life and its toxicity, as well as the degree of pain or inflammation of the joint process. The possible interaction with other drugs which the patient may be taking must be taken into account. It is advisable to use a specific preparation during two weeks at suitable doses before considering it ineffective and changing to a different drug. The use of two different NSAIDs does not add efficacy to the treatment and implies a greater risk of side effects.

Since the introduction of cortisone for the treatment of RA almost 50 years ago, glucocorticoids have been widely used for said purpose. After the initial enthusiasm due to their beneficial effects, the use decreased due to the high frequency of important side effects and the lack of evidence that the disease went into remission with their use. Their use being restricted due to these reasons for years, they are currently used more frequently given their clear anti-inflammatory action and the evidence, according to recent studies, suggesting that their use at low doses decreases the occurrence of erosions. Their use in RA must be reserved for those patients in whom the short-term benefits are greater than the risks of their long-term use, and always as part of a programmed treatment. Glucocorticoids are currently being questioned due to the fact that they seem to be related to an increase in the incidence of cardiac diseases in patients treated with these drugs.

Another large group of anti-inflammatories integrated by drugs which do not serve for treating pain in a determined moment, but which act by making the long-term activity of the disease lower, are disease-modifying anti-rheumatic drugs, slow-action remission inducers, immunosuppressants and cytotoxics. The use of these drugs is empirical and although all of them have shown their efficacy in the treatment of RA, long-term studies show discouraging results with regard to their benefit/toxicity ratio. They take weeks and even months in taking effect. There is no unanimous criterion concerning which treatment regimen to use or which drug or drug combination to choose. They are not effective in 100% of the patients, thus it is usual that the physician has to sequentially prescribe several of them until finding which one is the most effective and best tolerated. In this group are methotrexate, gold salts, chloroquine, sulfasalazine, D-penicillamine, azathioprine, cyclosporine...etc. They generally require control by the rheumatologist and close collaboration of the patient. When a drug of this type is prescribed, in addition to the degree of activity of the disease, the possible results which are expected to be obtained and the potential toxic effects thereof must be taken into account.

Therefore, there is the need to find compounds with a good anti-inflammatory activity and which in turn have scarce or zero side effects due to their cytotoxicity, thus allowing for long-term treatments.

The use of polyphenolic substances of a natural origin has resulted in combining a low toxicity with interesting antioxidant and anti-inflammatory properties, in addition to being moderate cyclooxygenase inhibitors.

Specifically, the 2,3-DHNA (apigenin) glycosides present in numerous plants (and particularly in chamomile) have shown to be important anti-inflammatory agents of a natural origin. The effectiveness of chamomile in the relief of symptoms of gastritis, gastric ulcers and other inflammatory processes in mucosae is due to the anti-inflammatory properties which are provided thereto by the 2,3-DHNA glycosides it contains (Merfort et al., 1994).

Some recent studies have shown that 2,3-DHNA could be effective in the treatment of inflammatory processes in the skin induced by free radicals (such as UV, X or γ irradiation, or chemical agents). Intradermal applications of liposomal 2,3-dehydronaringenin-7-glycoside inhibit the cutaneous inflammation caused by xanthine oxidase and cumene hydroperoxide in a dose-dependent manner. These results are in accordance with the properties as in-vitro scavengers of peroxide radicals and of the 2,3-DHNA superoxide anion, and which indicate that their antioxidant properties contribute to their anti-inflammatory effect in model systems (Fuchts and Milbradt, 1993). Studies carried out about the penetration into the skin of 2,3-DHNA, luteolin and 2,3-dehydronaringenin-7-glycoside have concluded that these flavonoids are not only absorbed in the surface of the skin, but they also penetrate in the deep layers of the skin. This is very important for their topical application as an antiphlogistic agent (Mefort et al., 1994).

Although there are several publications disclosing the use of 2,3-DHNA (apigenin) in pharmaceutical compositions for the treatment of certain diseases such as colon cancer (Walle and Halushka, 2001), diabetes (Ziegler, 2000), hepatic cholestasis (Gebhardt, 2001) or as an anxiolytic (Cassels et al., 1999), there is no publication in which the effectiveness of the use of 2,3-DHNA for the treatment of chronic diseases of an inflammatory character has been disclosed.

### DESCRIPTION OF THE INVENTION

A group of compounds has now been found which solves the drawback of the lack of compounds with scarce or zero cytotoxicity, with a good anti-inflammatory activity in inflammatory processes and/or chronic diseases generated by them, which furthermore allows for a prolonged administration to the patient. Therefore, according to a first aspect, the present invention refers to the use of compounds of Formula (I) 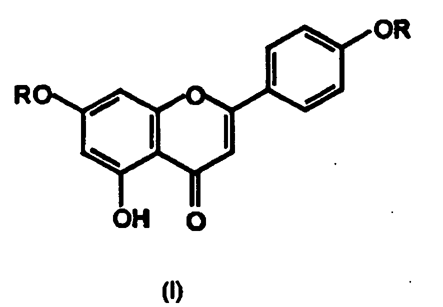 wherein the radicals R is a cation of an alkaline metal in the preparation of a drug for the treatment or prophylaxis of chronic diseases derived from inflammatory processes.

According to a preferred embodiment of the invention, in the compound of Formula (I), R is a potassium cation.

The compounds of Formula (I) have demonstrated a special efficacy in the treatment of rheumatoid arthritis. Therefore, according to a preferred embodiment of the invention, the latter refers to the use of compounds of Formula (I) in the preparation of a drug for the treatment of rheumatoid arthritis.

Below, the invention will be additionally described in more detail by means of a series of examples and drawings, merely as an illustrative example.
Figure 1 shows a graph of the variation of the extent of the inflammation of a paw of a mouse over time, caused by intradermal injection of carragenin.
Figure 2 shows a graph of the variation of the extent of the inflammation of a paw of a mouse over time, caused by the intraperitoneal administration of the compound of Formula (I).
Figure 3 shows a microscopic image of the subplantar region corresponding to degree 0 (HE 125X).
Figure 4a shows a microscopic image of the subplantar region corresponding to inflammatory phenomena (degree 1) characterized by dilated vessels and congestives with extensive areas of edema and small accumulations of preferably perivascular polymorphonuclear infiltrates (HE 125X).
Figure 4b shows a detail of the edematous phenomena and perivascular infiltrates (HE 200X).
Figure 5a shows a microscopic image with extensive areas of edema and numerous polymorphonuclear leukocyte infiltrates preferably arranged in a diffuse manner (HE 500X).
Figure 5b shows a detail of the areas of edema occupying the entire image, as well as of the extensive polymorphonuclear infiltrates (HE 312'SX).
Figure 6a shows a microscopic image of the subplantar cutaneous area with pronounced edematous phenomena and predominance of diffusely arranged polymorphonuclear infiltrates (HE 312'5X).
Figure 6b shows a detail of extensive polymorphonuclear infiltrates separating the muscular bundles (HE 500X).

### EXAMPLES

The anti-inflammatory capacity of the product of Formula (I), wherein R is a potassium cation, was tested: 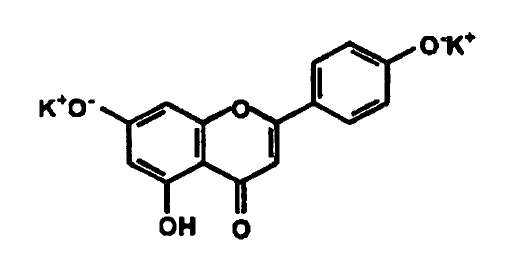 in male Swiss albino mice of 8 to 10 weeks of age (38-44 g), from the Servicio de Animales de Laboratorio (Laboratory Animals Department) of the University of Murcia. The animals were maintained under standard conditions (temperature of 22 ± 2°C, humidity 60 ± 4% and light/dark cycle of 12 hours) with food and drink ad libitum. The animals were anesthetized with ether in the moment of inducing inflammation and during the measurements of the volume of the paw.

### Test of paw edema in mice induced by carragenin

The inflammation was caused by intradermal injection of 0.05 ml of 1% Lambda Carrageenan (Sigma-Aldrich, Madrid, Spain) in saline solution in the plantar pad of the right hind paw. The measurement of the edema was carried out with a digital calibrator (Proinsa, Vitoria, Spain), measuring the 3 largest diameters of the paw for the volume calculation. The measurements were carried out in triplicate. The volume of the paw was measured immediately before (0 hours) and at 1, 5 and 24 hours after causing inflammation. The compound of Formula (I) was dissolved in sterile saline solution and intraperitoneally administered (70 mg/kg) 1 h before causing inflammation. The control group did not receive treatment. Groups of 10 animals were used.

The analysis of reliability of the measurement method used (digital electronic calibrator) has clearly shown that the measurements carried out in triplicate are significantly reliable (p<0.005).

### Volume evolution and inflammation inhibition

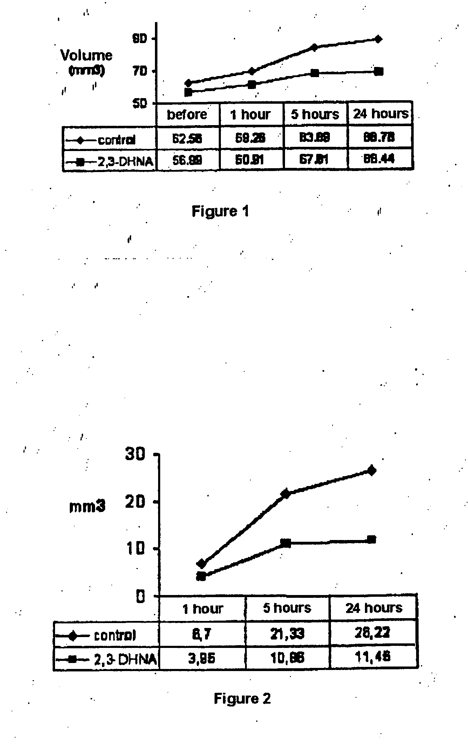

The intradermal injection of carragenin in the subplantar region of the paw in mice caused an increase of their volume from the first hour post-inoculation both in the control and in the treated group, although without showing significant differences with regard to 0 hours. The highest increase occurred at 5 hours in both groups, with significant differences with regard to 0 hours (p<0.005) and 1 hour post-inoculation (p<0.025). At 24 hours, the volume increase was lower, with significant differences both in the control and in the treated group, with regard to the values at 0 hours (p<0.005) and 1 hour post-inoculation (p<0.025) (Figure 1).

The compound of Formula (I) was intraperitoneally administered at a dose of 70 mg/kg one hour before the intradermal injection of carragenin in the subplantar region of the paw. Increases in the volume of inoculated paw were lower in the treated group than in the control group, significant differences existing after one hour (p<0.025), at 5 hours (p<0.005) and at 24 hours (p<0.0005) (Figure 2).

### Microscopic study and assessment

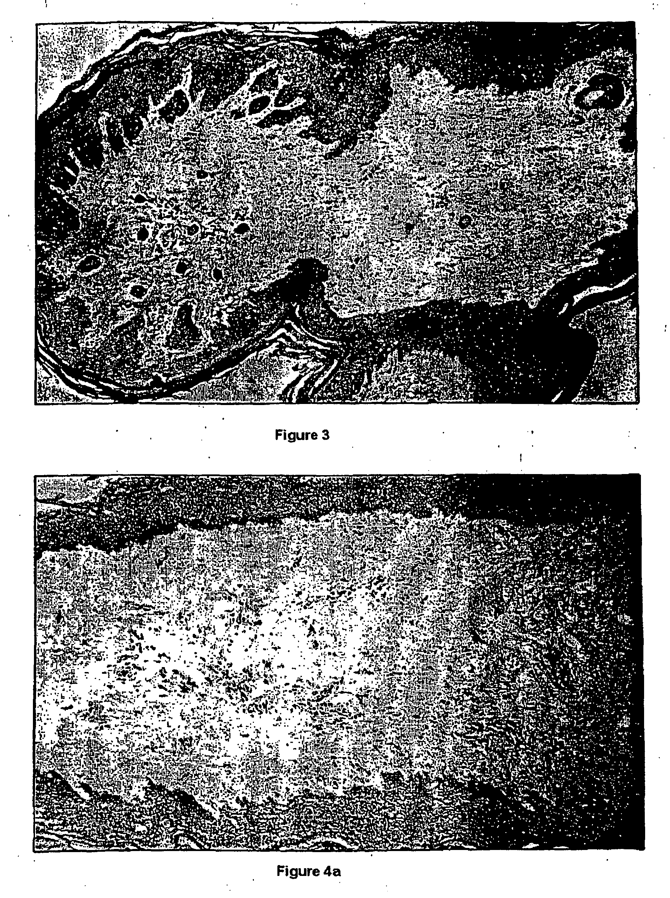

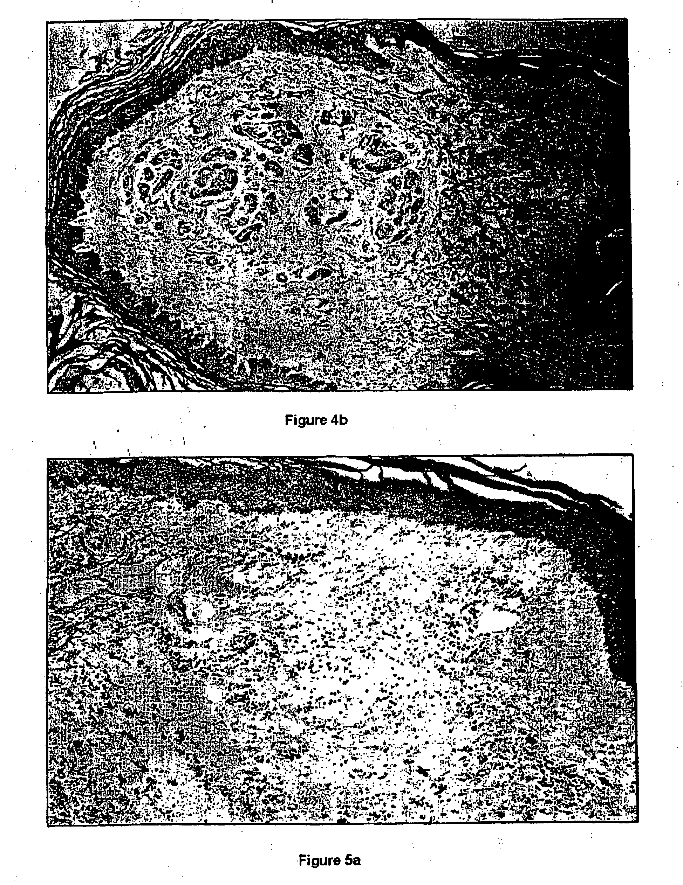

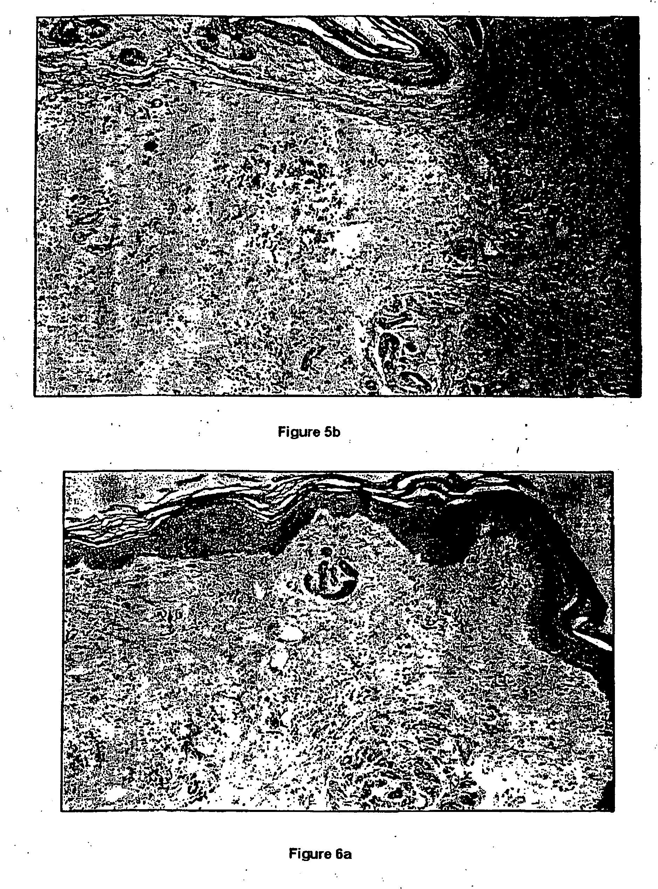

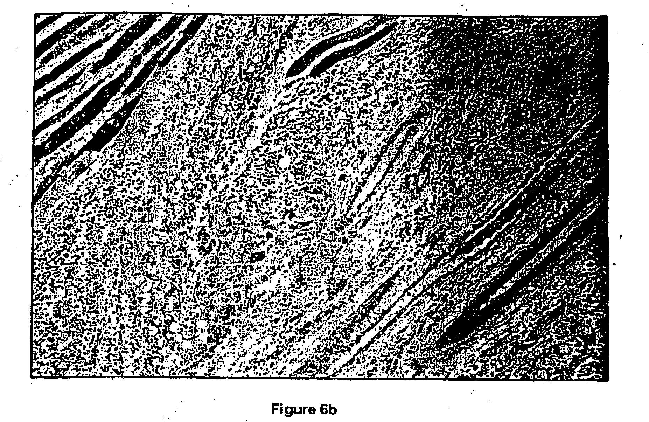

The plantar pad area injected was extirpated at 24 hours post-inoculation; fixed in 10% buffered neutral formaldehyde, included in paraffin by the standard method, sectioned at 3 µm and stained with hematoxilin-eosin for its optical study which was carried out by two observers. The presence of edema and polynuclear neutrophils and monocytes was measured, being assessed as 0 (absence), 1 (slight), 2 (moderate) and 3 (intense) (Figures 3 to 6b).

The results of the microscopic study clearly show the anti-inflammatory activity of the treatment with 2,3-DHNA. At 24 hours in 60% of the samples of the treated group, neither the presence of edema nor inflammatory infiltrate was observed (degree 0); whereas 26.65% showed a slight inflammation (degree 1) and 13.3% moderate (degree 2). In the control group (untreated), 53.3% of the samples showed an intense inflammation (degree 3) and 46.7% moderate (degree 2).

### Statistical analysis

A two-way repeated measures analysis of variance, complemented with a two-by-two test of equality of means by means of the Student's t-test, was carried out.

For the analysis of the reliability of the measurement method used, the interclass correlation coefficient has been applied.

### Immunochemical assay: TNFα measurement

For the immunochemical analyses, blood was extracted from the studied animals after 24 h of treatment, by means of cardiac puncture in Eppendorf tubes without heparin. It was maintained for 12 h at 2-8°C and was centrifuged at 2000 g. Serum aliquots were taken and frozen at -80°C. The TNFα measurement was subsequently carried out using an immunoassay enzyme kit provided by the R&D Systems company (catalogue number MTA00).

The technique used is based on that of the quantitative sandwich enzyme-immunoassay. Microplates covered with an affinity-purified, polyclonal, anti-TNFα antibody were used. The test serum (in addition to known quantities of a TNFα standard for the calibration line, provided with the kit) is incubated in the microplates. The unbound material is eliminated by washing and the complex is again incubated with a new polyclonal, TNFα-specific covalently bound to peroxidase. The quantity of bound peroxidase is measured by reaction with a chromogenic substrate and is proportional to the quantity of TNFα present in the test serum sample.

The obtained TNFα values for the control animals and those treated with 2,3-DHNA (Table 1) confirm the results shown by the inflammation assay and by the microscopic study.

**Table 1**

| | **TNFα (pmol/ml)** |
|---|---|
| **Control Group** | **143.07** |
| **Group treated with 2,3-DHNA** | **54.89** |

### Administration method

For the intraperitoneal administration of 2,3-DHNA in humans, an injectable composition is designed, to assure the presence of the compound in the bloodstream, composed of: sterile saline solution, monopotassium phosphate, potassium hydroxide, propylene glycol and benzyl alcohol according to the description given in Example 1.

### Example 1.

**Table 2**

| **Injectable composition** | **% by weight** |
|---|---|
| 2,3-DHNA | 0.30% |
| Sterile saline solution | 88.55% |
| Potassium phosphate | 0.10% |
| Potassium hydroxide | 0.05% |
| Propylene glycol | 10.00% |
| Benzyl alcohol | 1.00% |

## Claims

1. Use of compounds of formula 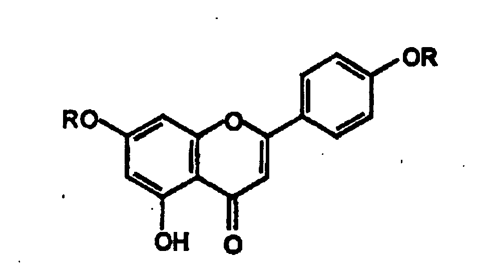 wherein the radical R is a cation of an alkaline metal, in the preparation of a drug for the treatment of chronic diseases derived from inflammatory processes.

2. Use according to claim 1, **characterized in that** said alkaline metal is a potassium cation.

3. Use according to any of claims 1 or 2, **characterized in that** said chronic disease is rheumatoid arthritis.

## Patentansprüche

1. Verwendung einer Verbindung der Formel 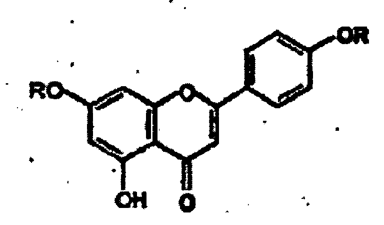 wobei das Radikal R ein Kation eines Alkalimetalls ist, bei der Herstellung, eines Medikaments für die Behandlung chronischer Krankheiten, die aus Entzündungsvorgängen herrühren.

2. Verwendung nach Anspruch 1, **dadurch gekennzeichnet, dass** das Alkalimetall ein Kaliumkation ist.

3. Verwendung nach einem der Ansprüche 1 oder 2, **dadurch gekennzeichnet, dass** die chronische Krankheit die rheumatoide Arthritis ist.

## Revendications

1. Utilisation de composés de formule : 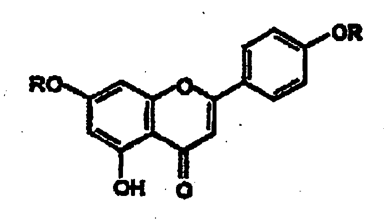 dans laquelle le radical R est un cation d'un métal alcalin, dans la préparation d'un médicament destiné au traitement de maladies chroniques dérivées de processus inflammatoires.

2. Utilisation selon la revendication 1, **caractérisée en ce que** ledit métal alcalin est un cation potassium.

3. Utilisation selon l'une quelconque des revendications 1 ou 2, **caractérisée en ce que** ladite maladie chronique est la polyarthrite rhumatoïde.
